# EUROPEAN PATENT SPECIFICATION

(11) **EP 0 689 819 B1**
(45) Date of publication and mention of the grant of the patent: **28.11.2001**
(21) Application number: 95304585.3
(22) Date of filing: 29.06.1995
(51) Int. Cl.: A61F 13/15

(54) **Method for making body fluids absorbent padding**
Verfahren zur Herstellung eines Körperflüssigkeiten absorbierenden Artikels
Procédé pour la fabrication d'un produit absorbant les fluides corporels

(30) Priority: 01.07.1994 JP 15112994
(43) Date of publication of application: 03.01.1996
(73) Proprietor: UNI-CHARM CORPORATION, Kawanoe-shi Ehime-ken (JP)
(72) Inventor: Wada, Ichiro, Kawanoe-shi, Ehime-ken (JP)
(74) Representative: Murgatroyd, Susan Elizabeth

(56) References cited:
- EP-A- 0 360 929
- GB-A- 2 258 840
- US-A- 4 636 417
- US-A- 4 895 749

## Description

The present invention relates to a method for making body fluids absorbent padding and more particularly, to a method for making body fluids absorbent padding such as a sanitary napkin, a menstruation pad, an incontinent pad, a disposable diaper and the like.

It is well known for making body fluids absorbent padding such as sanitary napkins and disposable diapers to bond a liquid-permeable topsheet made of thermoplastic synthetic fibers or a plastic film integrally to a liquid-absorbent core containing thermoplastic synthetic fibers by thermally embossing them. For example, Japanese Laid-Open Utility Model Application No. 1982-139318 discloses a technique by which a topsheet and at least an upper surface of a liquid-absorbent core of a disposable diaper are provided with thermal meltability and they are welded together. According to this technique, with the disposable diaper put on a wearer's body, the topsheet is never separated from the core and therefore body fluids discharged onto the topsheet are rapidly guided to the core under a capillary action so as to keep a skin-contacting surface of the topsheet dry.

In general, the liquid-absorbent core is primarily made of crushed pulp fibers and may be mixed with suitable thermoplastic synthetic fibers to provide the core with desired thermal meltability. The topsheet may be made of a nonwoven fabric of thermoplastic synthetic fibers or a plastic film to provide the top sheet also with desired thermal meltability. However, both the inter-fiber welding and the fiber/film welding can be achieved only by embossing at relatively high temperature and pressure and the regions of a disposable diaper subjected to such embossing disadvantageously form a thick and hard layer which may uncomfortably stimulate a wearer's skin.

In GB-A-2258840, a topsheet for use in a sanitary absorbent article comprises liquid guide capillary tubes extending downward from the top openings formed in the topsheet and having bottom openings surrounded by an irregularly undulating peripheral edge. The capillary tubes are formed by placing the topsheet on a mold having a pattern of openings formed therein and heating the topsheet under pressure so that parts of the topsheet swell into the mold openings and burst.

It is an object of the present invention to provide an improved method of making body fluid absorbent padding by which the topsheet and the core are integral with each other.

Accordingly, the present invention consists in a method for making body fluids absorbent padding comprising a liquid-permeable topsheet made of a perforated plastic film, and a liquid-absorbent core made of at least fibrous materials and having an upper surface covered with said topsheet, the method comprising steps of:
a. thermally softening a thermoplastic film having upper and lower surfaces for the topsheet so that said film partially bulges and ruptures under a differential pressure of the fluid to form a plurality of liquid guiding passages extending from the upper surface toward the lower surface of said film and lower ends of the passages are formed with a plurality of irregular jags as the film is ruptured;
b. placing the film on the upper surface of the core with the lower ends of the passages facing the core;
   and being characterised by:-
c. at least partially heating the film placed on the upper surface of the core so that the jags are deformed and intertwined with the individual fibers of the upper surface of the core.

Preferably, the step of partially heating the film comprises a step of partially heating the film under pressure.

Preferably, the step of partially heating said film comprises a step of heat-embossing said film.

Preferably, the step of intertwining the jags with the core comprises a step of mechanically intertwining the jags with the individual fibers of the core and/or welding them together.

Preferably, the upper surface of the core on which the jags are intertwined with the individual fibers of the core comprises a fibrous layer having a fineness of 0.11 to 1.1 tex (1 to 10 deniers), a weight per unit area of 10 to 45g/m² and an apparent thickness of 1 to 10mm.

In the present invention "skin-contacting area" means an area with which a wearer's skin is contacted when the padding is worn.

According to the method for making body fluids absorbent padding as described above, the jagged lower ends of the respective liquid guiding passages could be mechanically intertwined with the individual fibers of the core without heating, but the step of partially heating the film placed on the core causes contraction as well as deformation of the jagged lower ends and realizes sufficiently complicated and dense intertwinement to keep the tcpsheet and the core integral with each other.

In the drawings:-
Fig. 1 is a perspective view showing a sanitary napkin as partially broken away;
Fig. 2 is a fragmentary sectional view in an enlarged scale taken along a line X - X in Fig. 1; and
Fig. 3 is a schematic diagram illustrating steps (A) through (C) for making a topsheet of a sanitary napkin.

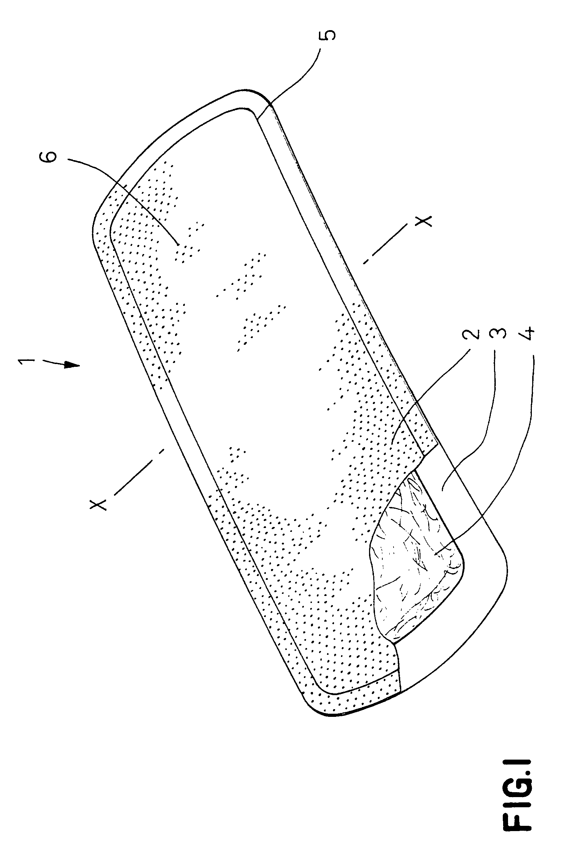

Referring to Fig. 1, a sanitary napkin 1 to be made according to the inventive method comprises a liquid-permeable topsheet 2 provided with a plurality of liquid guiding passages 6, a liquid-impermeable backsheet 3 and a liquid-absorbent core 4 disposed between these two sheets 2, 3. Portions of the top- and backsheets 2, 3 extending outward beyond a peripheral edge of the core 4 are watertightly bonded together along a bonding line 5.

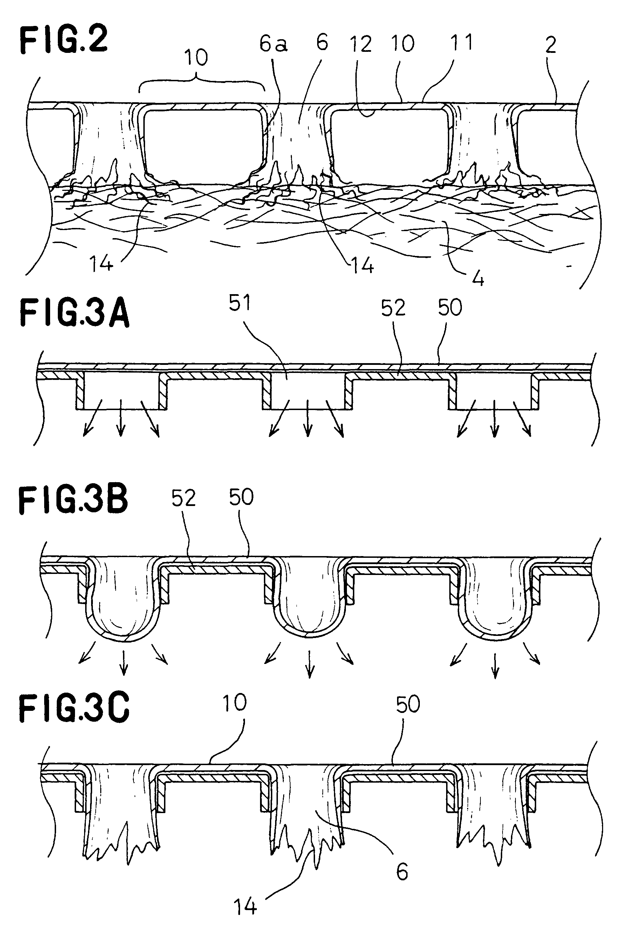

Referring to Fig. 2, the topsheet 2 is made of a polyethylene film and having a skin-contacting area 10 defined by a continuous upper surface 11 and liquid guiding passages 6 each having a sidewall 6a and extending through the topsheet 2 from the skin-contacting area 10 downward of a skin-noncontacting area 12 and arranged intermittently in a direction along the plane defined by the topsheet 2 (direction of X - Y of the topsheet). Each of the liquid guiding passages 6 may be tapered downward or diameter-enlarged downward or may be of a uniform diameter and has on a lower end of the sidewall 6a a plurality of irregular jags 14 which are formed by rupture of the film and thinner than a thickness of the sidewall 6a at its lower end. The core 4 comprises pulp fibers mixed or laminated with thermoplastic fibers of 0 to 30% by weight and high absorption polymer powders of 0 to 40% by weight. The thermoplastic fibers may be treated so as to become hydrophilic, if it is necessary. It is also possible to employ the core comprising a basic core formed of pulp fibers as primary material and covering the basic core with a tissue paper or netty sheet and a hydrophilic or hydrophobic fibrous layer disposed between the basic core and the topsheet 2. The fibrous layer, in general, may have a fineness of 0.11 to 1.1 tex (1 to 10 deniers), a weight per unit area of 10 to 45g/m² and an apparent thickness of 1 to 10mm. The fibrous layer may be integral with the basic core or merely placed on the basic core. The topsheet 2 and the core 4 are preferably in a relationship such that the jags 14 of the liquid guiding passages 6 are interposed among the individual fibers of the core 4 and at least mechanically intertwined with these individual fibers. More preferably, the jags 14 may be intertwined with the individual fibers of the core 4 by welding them together. Such reliable intertwinement of the topsheet 2 with the core 4 eliminates an apprehension that the topsheet 2 might be separated from the core 4 during handling or using the padding and allows body fluids discharged onto the topsheet to be rapidly guided to the core under the capillary action of the liquid guiding passages 6 followed by the capillary action of the fibers intertwined with the liquid guiding passages 6.

Referring to Fig. 3(A), a thermally softened plastic film 50 is placed on a die plate 52 having a plurality of fine perforations 51 and subjected to vacuum functioning to suck the film 50 downward with respect to the die plate 52. Referring to Fig. 3(B), the film bulges like balloons downward through the perforations 51 under the suction. Referring to Fig. 3(C), the lower end of each balloon-like bulge ruptures to form the liquid guiding passages 6 and a plurality of thin, irregular jags 14 and simultaneously the skin-contacting area 10 corresponding to the flat zone of the die plate 52. The film 50 thus molded may be separated from the die plate 52, cut into a desired dimension and then placed on the liquid-absorbent core 4 as shown by Fig. 2 or placed on the liquid-absorbent core 4 prior to cutting. Following these steps, the topsheet 2 is obtained. In any case, the topsheet 2 placed on the core 4 is heated and thereby the jags 14 are thermally contracted in order to stabilize the mechanical intertwinement of the jags 14 of the topsheet 2 and the individual fibers of the core 4. The jags 14 formed by extension of the film 50 under the action of vacuum are contracted and crimped as the film 50 is heated substantially to its plasticization temperature and, during this process, intertwined with the fibers of the core 4. If it is desired to enhance such intertwinement, the topsheet 2 and the core 4 may be embossed together with a heated embossing roll. The topsheet 2 thus treated produces a debossed pattern on its upper surface. In this case a relatively small embossing pressure is sufficient to weld the jags 14 to the core 4, since the jags 14 are thinner than the sidewall 6a of the liquid guiding passages 6 and correspondingly molten sooner than the liquid guiding passages 6. The relatively small embossing pressure can advantageously avoid an apprehension that the embossed spots might form thick and hard film.

With the method for making body fluids absorbent padding according to the invention, the jags formed on the lower ends of the liquid guiding passages extending through the topsheet are intertwined with the individual fibers of the liquid-absorbent core so that the topsheet and the core may be integrally bonded without loss of their softness and thereby a padding comfortable to wear may be obtained.

Furthermore, the surface of the core over which the jags are intertwined with the individual fibers of the core comprises a fibrous layer having specified fineness, weight per unit area and apparent thickness so that the jags may be sufficiently interposed among the fibers of the core and the intertwinement may be enhanced.

## Claims

1. A method for making body fluids absorbent padding comprising a liquid-permeable topsheet (2) made of a perforated plastic film, and a liquid-absorbent core (4) made of at least fibrous materials and having an upper surface covered with said topsheet (2), said method comprising steps of:
a. thermally softening a thermoplastic film (50) having upper and lower surfaces for said topsheet (2) so that said film (50) partially bulges and ruptures under a differential pressure of the fluid to form a plurality of liquid guiding passages (6) extending from the upper surface toward the lower surface of said film (50) and lower ends of said passages (6) are formed with a plurality of irregular jags (14) as said film (50) is ruptured;
b. placing said film (50) on the upper surface of the core (4) with the lower ends of said passages (6) facing said core (4);
and being **characterised by**:-
c. at least partially heating the film (50) placed on the upper surface of the core (4) so that the jags (14) are deformed and intertwined with the individual fibers of the upper surface of said core (4).

2. A method according to Claim 1, wherein said step of partially heating said film (50) comprises a step of partially heating said film (50) under pressure.

3. A method according to Claim 1, wherein said step of partially heating said film (50) comprises a step of heat-embossing said film (50).

4. A method according to Claim 1 or 2, wherein said step of intertwining the jags (14) with said core (4) comprises a step of mechanically inter wining said jags (14) with the individual fibers of said core (4) and/or welding them together.

5. A method according to any preceding claim, wherein the upper surface of said core (4) on which said jags (14) are intertwined with the individual fibers of said core (4) comprises a fibrous layer having a fineness of 0.11 to 1.1 tex (1 to 10 deniers), a weight per unit area of 10 to 45g/m² and an apparent thickness of 1 to 10mm.

## Patentansprüche

1. Verfahren zur Herstellung von Körperflüssigkeit absorbierenden Einlagen, umfassend eine flüssigkeitsdurchlässige Oberschicht (2), hergestellt aus einer perforierten Plastikfolie, und einen flüssigkeitsabsorbierenden Kern (4), hergestellt zumindest aus Fasermaterialien, wobei der Kern eine obere Fläche aufweist, die mit der Oberschicht (2) bedeckt ist, wobei das Verfahren umfaßt:
a. thermisches Erweichen einer thermoplastischen Folie (50) mit einer oberen und einer unteren Fläche für die Oberschicht (2), so daß die Folie (50) unter einem Druckunterschied der Flüssigkeit teilweise ausbaucht und reißt, um eine Vielzahl von flüssigkeitsführenden Passagen (6) zu bilden, die sich von der oberen Fläche hin zur unteren Fläche der Folie (50) erstrecken, und wobei die unteren Enden der Passagen (6) mit einer Vielzahl an unregelmäßigen Zacken (14) ausgestattet sind, wenn die Folie (50) reißt;
b. das Auflegen der Folie (50) auf die obere Fläche des Kerns (4), wobei die unteren Enden der Passagen (6) zum Kern (4) gerichtet sind;
und **gekennzeichnet** ist
c. durch ein zumindest teilweises Erwärmen der Folie (50), die auf die obere Fläche des Kerns (4) gelegt ist, so daß die Zacken (14) verformt und mit den einzelnen Fasern der oberen Fläche des Kerns (4) verflochten werden.

2. Verfahren nach Anspruch 1, wobei der Schritt des teilweisen Erwärmens der Folie (50) einen Schritt des teilweisen Erwärmens der Folie (50) unter Druck umfaßt.

3. Verfahren nach Anspruch 1, wobei der Schritt des teilweisen Erwärmens der Folie (50) einen Schritt der Wärmeprägung der Folie (50) umfaßt.

4. Verfahren nach Anspruch 1 oder 2, wobei der Schritt des Verflechtens der Zacken (14) mit dem Kern (4) einen Schritt des mechanischen Verflechtens der Zacken (14) mit den einzelnen Fasern des Kerns (4) und/oder das Zusammenschweißen derselben umfaßt.

5. Verfahren nach einem der vorstehenden Ansprüche, wobei die obere Fläche des Kerns (4), auf der die Zacken (14) mit den einzelnen Fasern des Kerns (4) verflochten sind, eine Faserschicht mit einer Feinheit von 0,11 bis 1,1 Tex (1 bis 10 Denier), ein Gewicht pro Flächeneinheit von 10 bis 45 g/m² und eine scheinbare Dicke von 1 bis 10 mm umfaßt.

## Revendications

1. Procédé de fabrication d'un produit absorbant les fluides corporels comprenant une feuille supérieure perméable au liquide (2) réalisée en un film plastique perforé, et un noyau absorbant le liquide (4) réalisé au moins en matériau fibreux et ayant une surface supérieure couverte avec ladite feuille supérieure (2), ledit procédé comprenant les étapes de :
a. ramollir thermiquement un film thermoplastique (50) ayant des surfaces supérieure et inférieure pour ladite feuille supérieure (2) de telle sorte que ledit film (50) se gondole partiellement et se rompt sous une pression différentielle du fluide pour former une pluralité de passage de guidage de liquide (6) s'étendant à partir de la surface supérieure en direction de la surface inférieure dudit film (50), et des extrémités inférieures desdits passages (6) sont formées avec une pluralité de déchirures irrégulières (14) lorsque ledit film (50) est rompu ;
b. placer ledit film (50) sur la surface supérieure du noyau (4) avec les extrémités inférieures desdits passages (6) faisant face audit noyau (4) ;
et étant **caractérisé par** :
c. au moins partiellement chauffer le film (50) placé sur la surface supérieure du noyau (4) de telle sorte que les déchirures (14) sont déformées et entrelacées avec les fibres individuelles de la surface supérieure dudit noyau (4).

2. Procédé selon la revendication 1, dans lequel ladite étape de partiellement chauffer ledit film (50) comprend une étape de partiellement chauffer ledit film (50) sous pression.

3. Procédé selon la revendication 1, dans lequel ladite étape de partiellement chauffer ledit film (50) comprend une étape de gaufrer thermiquement ledit film (50).

4. Procédé selon la revendication 1 ou 2, dans lequel ladite étape d'entrelacer les déchirures (14) avec ledit noyau (4) comprend une étape de mécaniquement entrelacer les déchirures (14) avec des fibres individuelles dudit noyau (4) et/ou de les souder ensembles.

5. Procédé selon l'une quelconque des revendications précédentes, dans lequel ladite surface supérieure dudit noyau (4) sur laquelle lesdites déchirures (14) sont entrelacées avec les fibres individuelles dudit noyau (4) comprend une couche fibreuse ayant une finesse de 0,11 à 1,1 tex (1 à 10 deniers), un poids par unité de surface de 10 à 45 g/m² et une épaisseur apparente de 1 à : 0 mm.
